# EUROPEAN PATENT SPECIFICATION

(11) **EP 3 329 925 B1**
(45) Date of publication and mention of the grant of the patent: **13.01.2021**
(21) Application number: 16832944.9
(22) Date of filing: 29.07.2016
(51) Int. Cl.: A61K 31/7105, A61K 47/34, A61K 47/42, A61K 48/00, A61P 19/02, A61P 29/00, A61K 9/107, C12N 15/09, A61K 47/60, A61K 47/64, A61K 47/69, A61K 9/00

(54) **POLYION COMPLEX CAPABLE OF DELIVERING MRNA INTO LIVING BODY FOR TREATING ARTHROPATHY**
POLYION-KOMPLEX MIT MRNA-ABGABE IN EINEN LEBENDEN KÖRPER ZUR BEHANDLUNG VON ARTHROPATHIEN
COMPLEXE POLYIONIQUE PERMETTANT D'ADMINISTRER DE L'ARNM DANS UN CORPS VIVANT POUR LE TRAITEMENT DE L'ARTHROPATHIE

(30) Priority: 31.07.2015 JP 2015151564
(43) Date of publication of application: 06.06.2018
(73) Proprietor: NanoCarrier Co., Ltd., Chiba 277-0871 (JP)
(72) Inventor: ITAKA, Keiji, Tokyo 113-8654 (JP); OHBA, Shinsuke, Tokyo 113-8654 (JP); TEI, Yuichi, Tokyo 113-8654 (JP); KATAOKA, Kazunori, Tokyo 113-8654 (JP)
(74) Representative: Hoffmann Eitle
(86) International application number: PCT/JP2016/072322
(87) International publication number: WO 2017/022665

(56) References cited:
- EP-A1- 3 106 177
- WO-A1-2015/121924
- SARAH R. HERLOFSEN ET AL: "Brief Report: Importance of SOX8 for In Vitro Chondrogenic Differentiation of Human Mesenchymal Stromal Cells : Importance of Sox8 for In Vitro Chondrogenesis", STEM CELLS., vol. 32, no. 6, 23 May 2014 (2014-05-23), pages 1629-1635, XP055546072, US ISSN: 1066-5099, DOI: 10.1002/stem.1642
- Kanjiro Miyata ET AL: "Rational design of smart supramolecular assemblies for gene delivery: chemical challenges in the creation of artificial virusesw", Chem. Soc. Rev., 2012, 41, 2562-2574, 7 April 2012 (2012-04-07), pages 2562-2574, XP055545520, Retrieved from the Internet: URL:https://pubs.rsc.org/en/content/articl epdf/2012/cs/c1cs15258k?page=search [retrieved on 2019-01-22]
- HAILATI AINI ET AL: "Messenger RNA delivery of a cartilage-anabolic transcription factor as a disease-modifying strategy for osteoarthritis treatment", SCIENTIFIC REPORTS, vol. 6, no. 1, 5 January 2016 (2016-01-05) , XP055546260, DOI: 10.1038/srep18743
- UCHIDA, SATOSHI ET AL.: 'In Vivo Messenger RNA Introduction into the Central Nervous System Using Polyplex Nanomicelle' PLOS ONE vol. 8, no. 2, 13 February 2013, ISSN 1932-6203 pages 1 - 9, XP002729006
- BABA, MIYUKI ET AL.: 'Treatment of neurological disorders by introducing mRNA in vivo using polyplex nanomicelles' JOURNAL OF CONTROLLED RELEASE vol. 201, 17 January 2015, ISSN 0168-3659 pages 41 - 48, XP029197851
- UCHIDA, HIROKUNI ET AL.: 'Modulated Protonation of Side Chain Aminoethylene Repeats in N- Substituted Polyaspartamides Promotes mRNA Transfection' JOURNAL OF THE AMERICAN CHEMICAL SOCIETY vol. 136, 2014, ISSN 0002-7863 pages 12396 - 12405, XP055362756

### SEQUENCE LISTING

<110> Itaka, Keiji
<120> Polyioncomplex which can efficiently deliver mRNA into a body and method for treating or preventing arthropathy
<130> PA54-9001WO
<150> JP 2015-151564
   <151> 2015-07-31
<160> 2
<170> PatentIn version 3.5
<210> 1
   <211> 5967
   <212> DNA
   <213> Homo sapiens
<220>
   <221> CDS
   <222> (191)..(1633)
<400> 1
ctttgggcct cataaacaac cacagaacca caagttgggt agcctggcag tgtcagaagt        60
   ctgaacccag catagtggtc agcaggcagg acgaatcaca ctgaatgcaa accacagggt       120
   ttcgcagcgt ggtaaaagaa atcattgagt cccccgcctt cagaagaggg tgcattttca       180
   ggaggaagcg atg gct tca gac agc ata ttt gag tca ttt cct tcg tac          229
   cca cag tgc ttc atg aga gaa tgc ata ctt gga atg aat cct tct aga         277
   gac gtc cac gat gcc agc acg agc cgc cgc ttc acg ccg cct tcc acc         325
   gcg ctg agc cca ggc aag atg agc gag gcg ttg ccg ctg ggc gcc ccg         373
   gac gcc ggc gct gcc ctg gcc ggc aag ctg agg agc ggc gac cgc agc         421
   atg gtg gag gtg ctg gcc gac cac ccg ggc gag ctg gtg cgc acc gac         469
   agc ccc aac ttc ctc tgc tcc gtg ctg cct acg cac tgg cgc tgc aac         517
   aag acc ctg ccc atc gct ttc aag gtg gtg gcc cta ggg gat gtt cca         565
   gat ggc act ctg gtc act gtg atg gct ggc aat gat gaa aac tac tcg          613
   gct gag ctg aga aat gct acc gca gcc atg aag aac cag gtt gca aga          661
   ttt aat gac ctc agg ttt gtc ggt cga agt gga aga ggg aaa agc ttc          709
   act ctg acc atc act gtc ttc aca aac cca ccg caa gtc gcc acc tac          757
   cac aga gcc atc aaa atc aca gtg gat ggg ccc cga gaa cct cga aga          805
   cat cgg cag aaa cta gat gat cag acc aag ccc ggg agc ttg tcc ttt          853
   tcc gag cgg ctc agt gaa ctg gag cag ctg cgg cgc aca gcc atg agg          901
   gtc agc cca cac cac cca gcc ccc acg ccc aac cct cgt gcc tcc ctg          949
   aac cac tcc act gcc ttt aac cct cag cct cag agt cag atg cag gat          997
   aca agg cag atc caa cca tcc cca ccg tgg tcc tac gat cag tcc tac         1045
   caa tac ctg gga tcc att gcc tct cct tct gtg cac cca gca acg ccc         1093
   att tca cct gga cgt gcc agc ggc atg aca acc ctc tct gca gaa ctt         1141
   tcc agt cga ctc tca acg gca ccc gac ctg aca gcg ttc agc gac ccg         1189
   cgc cag ttc ccc gcg ctg ccc tcc atc tcc gac ccc cgc atg cac tat         1237
   cca ggc gcc ttc acc tac tcc ccg acg ccg gtc acc tcg ggc atc ggc         1285
   atc ggc atg tcg gcc atg ggc tcg gcc acg cgc tac cac acc tac ctg         1333
   ccg ccg ccc tac ccc ggc tcg tcg caa gcg cag gga ggc ccg ttc caa       1381
   gcc agc tcg ccc tcc tac cac ctg tac tac ggc gcc tcg gcc ggc tcc       1429
   tac cag ttc tcc atg gtg ggc ggc gag cgc tcg ccg ccg cgc atc ctg       1477
   ccg ccc tgc acc aac gcc tcc acc ggc tcc gcg ctg ctc aac ccc agc       1525
   ctc ccg aac cag agc gac gtg gtg gag gcc gag ggc agc cac agc aac       1573
   tcc ccc acc aac atg gcg ccc tcc gcg cgc ctg gag gag gcc gtg tgg       1621
   agg ccc tac tga ggcgccaggc ctggcccggc tgggccccgc gggccgccgc           1673
   cttcgcctcc gggcgcgcgg gcctcctgtt cgcgacaagc ccgccgggat cccgggccct     1733
   gggcccggcc accgtcctgg ggccgagggc gcccgacggc caggatctcg ctgtaggtca     1793
   ggcccgcgca gcctcctgcg cccagaagcc cacgccgccg ccgtctgctg gcgccccggc     1853
   cctcgcggag gtgtccgagg cgacgcacct cgagggtgtc cgccggcccc agcacccagg     1913
   ggacgcgctg gaaagcaaac aggaagattc ccggagggaa actgtgaatg cttctgattt     1973
   agcaatgctg tgaataaaaa gaaagatttt atacccttga cttaactttt taaccaagtt      2033
   gtttattcca aagagtgtgg aattttggtt ggggtggggg gagaggaggg atgcaactcg     2093
   ccctgtttgg catctaattc ttatttttaa tttttccgca ccttatcaat tgcaaaatgc     2153
   gtatttgcat ttgggtggtt tttattttta tatacgttta tataaatata tataaattga     2213
   gcttgcttct ttcttgcttt gaccatggaa agaaatatga ttcccttttc tttaagtttt      2273
   atttaacttt tcttttggac ttttgggtag ttgttttttt ttgttttgtt ttgttttttt      2333
   gagaaacagc tacagctttg ggtcattttt aactactgta ttcccacaag gaatccccag     2393
   atatttatgt atcttgatgt tcagacattt atgtgttgat aattttttaa ttatttaaat     2453
   gtacttatat taagaaaaat atcaagtact acattttctt ttgttcttga tagtagccaa     2513
   agttaaatgt atcacattga agaaggctag aaaaaaagaa tgagtaatgt gatcgcttgg     2573
   ttatccagaa gtattgttta cattaaactc cctttcatgt taatcaaaca agtgagtagc     2633
   tcacgcagca acgtttttaa taggattttt agacactgag ggtcactcca aggatcagaa 2693
   gtatggaatt ttctgccagg ctcaacaagg gtctcatatc taacttcctc cttaaaacag 2753
   agaaggtcaa tctagttcca gagggttgag gcaggtgcca ataattacat ctttggagag 2813
   gatttgattt ctgcccaggg atttgctcac cccaaggtca tctgataatt tcacagatgc 2873
   tgtgtaacag aacacagcca aagtaaactg tgtaggggag ccacatttac ataggaacca 2933
   aatcaatgaa tttaggggtt acgattatag caatttaagg gcccaccaga agcaggcctc 2993
   gaggagtcaa tttgcctctg tgtgcctcag tggagacaag tgggaaaaca tggtcccacc 3053
   tgtgcgagac cccctgtcct gtgctgctca ctcaacaaca tctttgtgtt gctttcacca 3113
   ggctgagacc ctaccctatg gggtatatgg gcttttacct gtgcaccagt gtgacaggaa 3173
   agattcatgt cactactgtc cgtggctaca attcaaaggt atccaatgtc gctgtaaatt 3233
   ttatggcact atttttattg gaggatttgg tcagaatgca gttgttgtac aactcataaa 3293
   tactaactgc tgattttgac acatgtgtgc tccaaatgat ctggtggtta tttaacgtac 3353
   ctcttaaaat tcgttgaaac gatttcaggt caactctgaa gagtatttga aagcaggact 3413
   tcagaacagt gtttgatttt tattttataa atttaagcat tcaaattagg caaatctttg 3473
   gctgcaggca gcaaaaacag ctggacttat ttaaaacaac ttgtttttga gttttcttat 3533
   atatatattg attatttgtt ttacacacat gcagtagcac tttggtaaga gttaaagagt 3593
   aaagcagctt atgttgtcag gtcgttctta tctagagaag agctatagca gatctcggac 3653
   aaactcagaa tatattcact ttcatttttg acaggattcc ctccacaact cagtttcata 3713
   tattattccg tattacattt ttgcagctaa attaccataa aatgtcagca aatgtaaaaa 3773
   tttaatttct gaaaagcacc attagcccat ttcccccaaa ttaaacgtaa atgttttttt 3833
   tcagcacatg ttaccatgtc tgacctgcaa aaatgctgga gaaaaatgaa ggaaaaaatt 3893
   atgtttttca gtttaattct gttaactgaa gatattccaa ctcaaaacca gcctcatgct 3953
   ctgattagat aatcttttac attgaacctt tactctcaaa gccatgtgtg gagggggctt 4013
   gtcactattg taggctcact ggattggtca tttagagttt cacagactct taccagcata 4073
   tatagtattt aattgtttca aaaaaaatca aactgtagtt gttttggcga taggtctcac 4133
   gcaacacatt tttgtatgtg tgtgtgtgtg cgtgtgtgtg tgtgtgtgtg aaaaattgca 4193
   ttcattgact tcaggtagat taaggtatct ttttattcat tgccctcagg aaagttaagg 4253
   tatcaatgag acccttaagc caatcatgta ataactgcat gtgtctggtc caggagaagt 4313
   attgaataag ccatttctac tgcttactca tgtccctatt tatgatttca acatggatac 4373
   atatttcagt tctttctttt tctcactatc tgaaaataca tttccctccc tctcttcccc 4433
   ccaatatctc cctttttttc tctcttcctc tatcttccaa accccacttt ctccctcctc 4493
   cttttcctgt gttctcttaa gcagatagca cataccccca cccagtacca aatttcagaa 4553
   cacaagaagg tccagttctt cccccttcac ataaaggaac atggtttgtc agcctttctc 4613
   ctgtttatgg gtttcttcca gcagaacaga gacattgcca accatattgg atctgcttgc 4673
   tgtccaaacc agcaaacttt cctgggcaaa tcacaatcag tgagtaaata gacagccttt 4733
   ctgctgcctt gggtttctgt gcagataaac agaaatgctc tgattagaaa ggaaatgaat 4793
   ggttccactc aaatgtcctg caatttagga ttgcagattt ctgccttgaa atacctgttt 4853
   ctttgggaca ttccgtcctg atgattttta tttttgttgg tttttatttt tggggggaat 4913
   gacatgtttg ggtcttttat acatgaaaat ttgtttgaca ataatctcac aaaacatatt 4973
   ttacatctga acaaaatgcc tttttgttta ccgtagcgta tacatttgtt ttgggatttt 5033
   tgtgtgtttg ttgggaattt tgtttttagc caggtcagta ttgatgaggc tgatcatttg 5093
   gctctttttt tccttccaga agagttgcat caacaaagtt aattgtattt atgtatgtaa 5153
   atagatttta agcttcatta taaaatattg ttaatgccta taactttttt tcaatttttt 5213
   tgtgtgtgtt tctaaggact ttttcttagg tttgctaaat actgtaggga aaaaaatgct 5273
   tctttctact ttgtttattt tagactttaa aatgagctac ttcttattca cttttgtaaa 5333
   cagctaatag catggttcca atttttttta agttcacttt ttttgttcta ggggaaatga 5393
   atgtgcaaaa aaagaaaaag aactgttggt tatttgtgtt attctggatg tataaaaatc 5453
   aatggaaaaa aataaacttt caaattgaaa tgacggtata acacatctac tgaaaaagca 5513
   acgggaaatg tggtcctatt taagccagcc cccacctagg gtctatttgt gtggcagtta 5573
   ttgggtttgg tcacaaaaca tcctgaaaat tcgtgcgtgg gcttctttct ccctggtaca 5633
   aacgtatgga atgcttctta aaggggaact gtcaagctgg tgtcttcagc cagatgacat 5693
   gagagaatat cccagaaccc tctctccaag gtgtttctag atagcacagg agagcaggca 5753
   ctgcactgtc cacagtccac ggtacacagt cgggtgggcc gcctcccctc tcctgggagc 5813
   attcgtcgtg cccagcctga gcagggcagc tggactgctg ctgttcagga gccaccagag 5873
   ccttcctctc tttgtaccac agtttcttct gtaaatccag tgttacaatc agtgtgaatg 5933
   gcaaataaac agtttgacaa gtacatacac cata 5967
<210> 2
   <211> 480
   <212> PRT
   <213> Homo sapiens
<400> 2

## Description

### Technical Field

The present invention relates to a polyion complex for use in a method of treating osteoarthritis or rheumatoid arthritis as defined in the appended claims.

### Background Art

Drug delivery systems for delivering drugs to a suitable site in a body have been researched and developed to provide a new medicament with few side effects. Among these systems, a drug delivery system using a polyion complex (hereinafter also referred to as "PIC") is drawing attention as a technique which can specifically deliver drugs to affected areas by including the drugs in nanomicelles.

As a technique applying PIC, in particular, a system which can deliver nucleic acids into a body with few side effects has been developed (Patent Literature 1 and 2). Inventors in Patent Literature 1 and 2 have developed a polyion complex of DNA and a new cationic polymer. A polyion complex of RNA and a cationic polymer has also been published by inventors (Non Patent Literature 1).

Osteoarthritis (OA) is a chronic degradative joint disease and is caused by collapse of balance between synthesis and degradation of the cartilage (Non Patent Literature 2). Osteoarthritis is a major health problem in the elderly (Non Patent Literature 3). However, disease-modifying drugs for osteoarthritis (DMOAD) for clinical use have not been developed yet (Non Patent Literature 4 and 5).

Runx1 is known to control chondrogenesis in embryos and adults (Non Patent Literature 6 and 7). Two different DMOAD candidate compounds have been proved to have therapeutic effects through Runx1 (Non Patent Literature 8 and 9).

Herlofsen, Sarah R., et al. describe the importance of SOX8 for *in vitro* chondrogenic differentiation of human mesenchymal stromal cells and demonstrate that SOX8 mRNA levels decrease during *in vitro* dedifferentiation of human articular chondrocytes and increase during chondrogenic differentiation of mesenchymal stroma cells (Herlofsen, Sarah R., et al. "Brief report: importance of SOX8 for in vitro chondrogenic differentiation of human mesenchymal stromal cells." Stem cells 32.6 (2014): 1629-1635).

Uchida, Satoshi, et al. describe *in vivo* messenger RNA introduction into the central nervous system using polyplex nanomicelle using a novel carrier based on self-assembly of a polyethylene glycol (PEG)-polyamino acid block copolymer, polyplex nanomicelle, to administer mRNA into the central nervous system (CNS) (Uchida, Satoshi, et al. "In vivo messenger RNA introduction into the central nervous system using polyplex nanomicelle." PloS one 8.2 (2013)).

Baba, Miyuki, et al describe the treatment of neurological disorders by introducing mRNA *in vivo* using a novel carrier based on the self-assembly of polyethylene glycol (PEG)-polyamino acid block copolymer, i.e. polyplex nanomicelles (Baba, Miyuki, et al. "Treatment of neurological disorders by introducing mRNA in vivo using polyplex nanomicelles." Journal of controlled release 201 (2015): 41-48).

### Citation List

### Patent Literature

Patent Literature 1: Japanese Patent No. 4535229
Patent Literature 2: Japanese Patent No. 5061349

### Non Patent Literature

Non Patent Literature 1: H. Uchida et al., Journal of the American Chemical Society 136, 12396-12405 (2014)
Non Patent Literature 2: G. Nuki, Z Rheumatol, 58, 142-147 (1999)
Non Patent Literature 3: D. T. Felson et al., Annals of Internal Medicine 133, 635-646 (2000)
Non Patent Literature 4: Z. Jotanovic et al., Current drug targets 15, 635-661 (2014)
Non Patent Literature 5: D. J. Hunter, Nat Rev Rheumatol 7, 13-22 (2011)
Non Patent Literature 6: A. Kimura et al., Development. (England, 2010), vol. 137, pp. 1159-1167
Non Patent Literature 7: K. T. LeBlanc et al., J. Cell. Physiol., 230 (2), pp. 440-448 (2015)
Non Patent Literature 8: F. Yano et al., Ann Rheum Dis 72, 748-753 (2013)
Non Patent Literature 9: K. Johnson et al., Science 336, 717-721 (2012)

### Summary of Invention

### Technical Problem

The present invention provides a polyion complex for use in a method of treating osteoarthritis or rheumatoid arthritis as defined in the appended claims.

### Solution to Problem

The present inventors proved that a polyion complex comprising a cationic polymer and mRNA, wherein the cationic polymer is a polymer comprising an unnatural amino acid as a monomer unit and the unnatural amino acid is an amino acid having a group represented by -(NH-(CH₂)₂)ₚ-NH₂, wherein p is 2, 3 or 4, as a side chain, highly expresses mRNA continuously for a long time. The present inventors have proved that osteoarthritis can be treated by administering the polyion complex comprising the cationic polymer and Runx1 mRNA. The present invention is based on these findings.

The present invention is defined in the appended claims.

Also disclosed is the following.
(1) a polyion complex comprising a cationic polymer and mRNA,
   wherein the cationic polymer is a polymer comprising a cationic unnatural amino acid as a monomer unit and the cationic unnatural amino acid is an amino acid having a group represented by - (NH-(CH₂)₂)ₚ-NH₂, wherein p is 2, 3 or 4, as a side chain.
(2) The polyion complex according to (1) above, wherein the cationic polymer is a block copolymer with polyethylene glycol.
(3) The polyion complex according to (1) or (2) above, wherein the cationic polymer is represented by the following general formula (I): 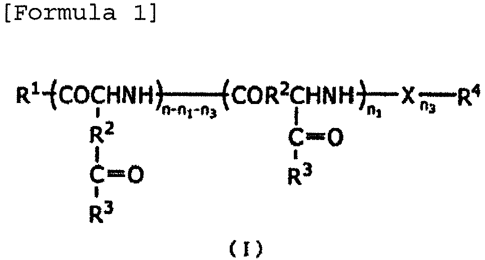 wherein
   R¹ is polyethylene glycol and may be linked to an amino acid neighboring the polyethylene glycol through a linker, R² is methylene or ethylene,
   R³ is a group represented by - (NH-(CH₂)₂)ₚ-NH₂, and p is 2, 3 or 4,
   R⁴ is hydrogen, a protecting group, a hydrophobic group, or a polymerizable group,
   X is a cationic natural amino acid,
   n is any integer from 2 to 5000,
   n₁ is any integer from 0 to 5000,
   n₃ is any integer from 0 to 5000,
   n - n₁ - n₃ is an integer equal to or greater than 0, and though the repeating units in the formula are shown in a particular order for convenience of description, the repeating units may be present in any order and the repeating units may be present at random, and each of the repeating units may be same or different.
(4) A pharmaceutical composition for treating arthropathy, comprising the polyion complex according to any one of (1) to (3) above,
   wherein the mRNA is a Runx1 mRNA.
(5) The pharmaceutical composition according to (4) above, wherein p is 3 or 4.
(6) The pharmaceutical composition according to (5) above, wherein p is 4.
(7) The pharmaceutical composition according to any one of (4) to (6) above, wherein the pharmaceutical composition is administered once every 2 days or once every 3 days.
(8) The pharmaceutical composition according to (5) or (6) above, wherein the pharmaceutical composition is administered once every 7 days.
(9) The pharmaceutical composition according to any one of (4) to (8) above, wherein the arthropathy is osteoarthritis or rheumatoid arthritis.
(10) A delivery agent for use in delivering mRNA into cells, comprising the polyion complex according to (3) above, wherein p is 3 or 4.
(11) The delivery agent according to (10) above, wherein p is 4.

### Brief Description of Drawings

[Figure 1] Figure 1 is photos showing the expression of luciferase mRNA intraarticularly administered.
[Figure 2] Figure 2 is graphs showing the transition over time of the expression level of luciferase mRNA intraarticularly administered.
[Figure 3] Figure 3 shows the effect of administration of Runx1 mRNA on osteoarthritis. A polymer in which p is 2 is used as a cationic polymer in Figure 3.
[Figure 4] Figure 4 shows the effect of administration of Runx1 mRNA on osteoarthritis. A polymer in which p is 3 is used as a cationic polymer in Figure 4.
[Figure 5] Figure 5 shows the expression of various marker genes and the effect on cell death examined by TUNEL method in group receiving administration of Runx1 mRNA.

### Description of Embodiment

The polyion complex employed in the present invention is used in a method of treating osteoarthritis or rheumatoid arthritis as defined in the appended claims and comprises at least (i) a block copolymer comprising a cationic polymer and (ii) mRNA. The polyion complex used in the present invention as defined in the appended claims is characterized in that the cationic polymer is a polymer comprising an unnatural amino acid as a monomer unit and the unnatural amino acid has a group represented by - (NH-(CH₂)₂)ₚ-NH₂, wherein p is 2, 3 or 4, as a side chain. P is preferably 3 or 4, and most preferably 3. The cationic polymer may be a cationic polymer forming a block copolymer with a polyethylene glycol block. (i) the copolymer and (ii) the mRNA form a polyion complex in a solution. The polyion complex used in the present invention as defined in the appended claims can be provided in a solution, preferably in an aqueous solution.

The polyion complex used in the present invention as defined in the appended claims comprises a cationic polymer and mRNA, and is thought to be in the form of nanomicelles. Therefore, the polyion complex used in the present invention may be referred to as a polyion complex-type micelle in the specification.

As used herein, mRNA refers to messenger RNA.

Cytidine and uridine in mRNA in the polvion complex used in the present invention may be modified. Examples of the modified cytidine include, for example, 5-methyl-cytidine, and examples of the modified uridine include pseudouridine and 2-thio-cytidine. The modified cytidine and uridine may comprise 10 mole % or more, 20 mole % or more, or 30 mole % or more of total cytidine and uridine.

As used herein, "a subject" is a mammal including a human. The subject may be a healthy subject or may be a subject suffering from some disease.

In the present invention as defined in the appended claims, the cationic polymer may comprise, for example, a cationic natural amino acid and/or a cationic unnatural amino acid as a monomer unit. Here, examples of the cationic natural amino acid preferably include histidine, tryptophane, ornithine, arginine and lysine, more preferably include arginine, ornithine and lysine, further preferably include ornithine and lysine, and further more preferably include lysine.

In the present invention as defined in the appended claims, the polymer part comprising an unnatural amino acid as a monomer unit in the cationic polymer can be obtained, for example, by introducing a group represented by -(NH-(CH₂)₂)ₚ-NH₂, wherein p is 2, 3 or 4, into a polymer comprising aspartic acid or glutamic acid as a monomer unit. Those skilled in the art can obtain this polymer part comprising the unnatural amino acid as a monomer unit by reacting a polymer comprising aspartic acid or glutamic acid as a monomer unit with diethyltriamine, triethyltetraamine or tetraethylpentaamine.

In the preferred embodiment of the present invention, the cationic polymer can be a polymer represented by the following general formula (I): 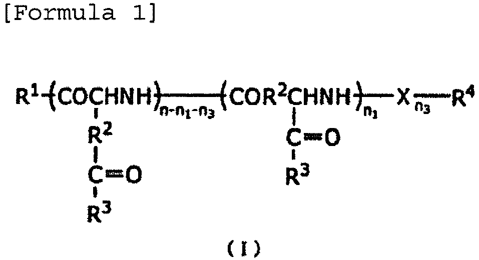 wherein
R¹ is polyethylene glycol and may be linked to an amino acid neighboring the polyethylene glycol through a linker,
R² is methylene or ethylene,
R³ is a group represented by -(NH-(CH₂)₂)ₚ-NH₂, and p is 2, 3 or 4,
R⁴ is hydrogen, a protecting group, a hydrophobic group, or a polymerizable group,
X is a cationic amino acid,
n is any integer from 2 to 5000,
n₁ is any integer from 0 to 5000,
n₃ is any integer from 0 to 5000,
n - n₁ - n₃ is an integer equal to or greater than 0, and though the repeating units in the formula are shown in a particular order for convenience of description, the repeating units may be present in any order and the repeating units may be present at random, and the repeating units may be same or different.

In the formula (I) above, p is preferably 3 or 4, and most preferably 3.

In the formula (I) above, examples of the protecting group include C₁₋₆ alkylcarbonyl groups, and the protecting group is preferably an acetyl group, and examples of the hydrophobic group include benzene, naphthalene, anthracene, pyrene and derivatives thereof or C₁₋₆ alkyl groups, and examples of the polymerizable group include a methacryloyl group and an acryloyl group. Methods for introducing the protecting group, the hydrophobic group and the polymerizable group into a block copolymer are well-known to those skilled in the art.

In the formula (I) above, though the average degree of polymerization of polyethylene glycol (PEG) is 5 to 20000, preferably 10 to 5000, and more preferably 40 to 500, the degree is not particularly limited as long as the polyion complex formation of the block copolymer and mRNA is not inhibited. The end of PEG of the cationic polymer may be protected by a hydroxy group, a methoxy group or a protecting group.

In the formula (I) above, the linker may be for example -(CH₂)r-NH-, wherein r is any integer from 1 to 5, or -(CH₂)s-CO-, wherein s is any integer from 1 to 5, and may be linked to the neighboring amino acid in the formula (I) preferably through a peptide bond. The linker may be preferably linked to PEG on the methylene side of PEG through an O atom of PEG.

In the formula (I) above, n is any integer from 0 to 5000, for example, any integer from 0 to 500; m is any integer from 0 to 5000, for example, any integer from 0 to 500; and m + n is any integer from 2 to 5000, for example, any integer from 2 to 500.

In one embodiment of the present invention as defined in the appended claims the cationic polymer may be a block copolymer of a PEG-linker-polycation block, where the PEG, the linker and the polycation block are as defined above.

The present inventors have found that when the cationic polymer used for the formation of a polyion complex is a polymer comprising a monomer unit having a group represented by -(NH-(CH₂)₂)ₚ-NH₂, wherein p is 2, 3 or 4, as a side chain, mRNA can be present in a body for a long time, and that, in particular, when p is 3 or 4, mRNA is highly expressed continuously for a considerably long time in a body. Therefore, in the present invention, the cationic polymer is a cationic polymer comprising a monomer unit having a group represented by -(NH-(CH₂)₂)ₚ-NH₂, wherein p is 2, 3 or 4, as a side chain, wherein p is more preferably 3 or 4, and further preferably 3.

The present inventors administered the polyion complex as defined in the claims using a Runx1 mRNA as the mRNA in the polyion complex to arthropathy model animals and have found that the progress of symptoms of arthropathy was slowed or the symptoms were improved by Runx1.

Therefore, in the present invention, a pharmaceutical composition for use in treating osteoarthritis or rheumatoid arthritis as defined in the appended claims comprising the polyion complex of the present invention is provided. The mRNA is an mRNA of a factor promoting joint formation selected from transcription factors promoting joint formation, such as Runt-related transcription factor 1 (Runx1), core-binding factor beta (Cbfbeta), sex-determining box 9 (Sox9) and Proteoglycan 4.

In one embodiment of the present invention, the pharmaceutical composition is a pharmaceutical composition for use in treating osteoarthritis or rheumatoid arthritis, comprising polyion complex-type micelles of a cationic polymer and a Runx1 mRNA, wherein R¹ in the formula (I) is protected polyethylene glycol, and p is 3 or 4, and R⁴ is hydrogen or a protecting group.

In one embodiment, the pharmaceutical composition used in the present invention may further comprise an excipient.

In one embodiment, the pharmaceutical composition used in the present invention may be formulated for intraarticular administration.

In another aspect of the present disclosure, there is provided a method for treating arthropathy in a subject in need thereof, comprising administering the effective amount of the pharmaceutical composition used in the present invention to a subject.

In the present disclosure the administration of the pharmaceutical composition used in the present invention to a subject allows the expression of a protein from mRNA to be sustained in the subject at least for 2 days, preferably for 3 days. Therefore, the pharmaceutical composition used in the present invention can be administered once every 2 days or once every 3 days.

When p is 3 or 4 in the group represented by -(NH-(CH₂)₂)ₚ-NH₂, mRNA can be expressed in a subject at least for 4 days, preferably for 8 days. Therefore, when p is 3 or 4 in the group represented by - (NH- (CH₂)₂)ₚ-NH₂, the pharmaceutical composition used in the present invention can be administered, for example, once every 3 days, preferably once every 4 days, more preferably once every 5 days, further preferably once every 6 days, and further more preferably once every week. Thereby, frequency of administration is decreased and a patient's burden of treatment is greatly reduced.

### Examples

### Example 1: Production of a polyion complex

First, a polyion complex containing mRNA was produced.

### 1-1. Synthesis of a polymer comprising pAsp (DET), pAsp (TET) or pAsp (TEP) as a monomer unit

In this Example, the group represented by -(NH-(CH₂)₂)ₚ-NH₂, wherein p is 2, 3 or 4, was introduced to a side chain of polyaspartic acid to obtain the title polymer. Specifically, polyethylene glycol with a number average molecular weight of 23,000 having a methoxy group at one end and an aminopropyl group at the other end (MeO-PEG-NH₂) (purchased from NOF CORPORATION) was dissolved in methylene chloride. β-benzyl-L-aspartate-N-carboxy anhydride (BLA-NCA) (purchased from Chuo Kaseihin Co., Inc.) was added to and dissolved in a mixed solution of N,N-dimethylformamide (DMF) and the methylene chloride solution to obtain a reaction solution. Then, the reaction solution was reacted at 40°C for 2 days to obtain polyethylene glycol - poly (β-benzyl -L-aspartate) block copolymer (MeO-PEG-PBLA). Analysis by ¹H-NMR showed that the number average molecular weight of the PBLA moiety was about 14,000 and the degree of polymerisation was about 66.

Next, fractions of MeO-PEG-PBLA were reacted with diethylenetriamine, triethyltetraamine and tetraethylpentaamine, respectively, to obtain MeO-PEG-pAsp (DET) block copolymer, MeO-PEG-pAsp (TET) block copolymer, and MeO-PEG-pAsp (TEP) block copolymer. Specifically, MeO-PEG-PBLA was dissolved in benzene and the resultant was freeze-dried. The freeze-dried MeO-PEG-PBLA was dissolved in N,N-dimethylformamide (DMF). Thereafter, diethylenetriamine, triethyltetraamine and tetraethylpentaamine (all purchased from Wako Pure Chemical Industries, Ltd.) were dropped to the obtained solutions, respectively, and the mixtures were reacted under mild anhydrous conditions of 40°C to obtain MeO-PEG-pAsp (DET) block copolymer, MeO-PEG-pAsp (TET) block copolymer, and MeO-PEG-pAsp (TEP) block copolymer. The obtained block copolymers showed sharp single molecular weight distribution (Mw/Mn = 1.04) in gel permeation chromatography (GPC).

### 1-2. Preparation of mRNA

First, human RUNX1 expression vectors were prepared. An open reading frame of 3×FLAG tagged human RUNX1 (GenBank registration number: NM_001754.4; SEQ ID NO: 1) flanked with XhoI and BaII/SmaI restriction enzyme cleavage sites (hereinafter, referred to as RUNX1-FL) was cloned into pUC57 vectors. The obtained RUNX1-FLs were introduced into pSP17 vectors. Driven by T7 promoters, human RUNX1-FLs were transcribed from lineared pSP17 vectors using mMESSAGE mMACHINE T7 Ultra Kit (Ambion, Carlsbad, CA, USA), and then poly A was added to the transcripts using a poly (A) tail kit (Ambion). The transcribed mRNAs were purified using RNeasy Mini Kit (Qiagen, Hilden, Germany). EGFP mRNA was prepared in the same way. Luc2 mRNA was prepared using the transcriptional region of a protein of pGL4.13 (Promega, Madison, WI, USA) in the same way.

### 1-3. Preparation of polyion complex-type micelles

Each of the copolymer solutions obtained in 1-1 and various mRNA solutions were mixed separately in 10 mM Hepes buffer (pH7.3) to obtain polyion complexes. The concentration of mRNAs was 225 µg/mL, and the mixing ratio (N/P) of an amino group (N) of amino acid residues in the copolymers and a phosphoric acid group (P) in nucleic acids was 8. Because the copolymer solutions and the mRNA solutions were mixed in a quantitative ratio of 1:2, the concentration of mRNA became 150 µg/mL.

As a control, a lipoplex of mRNA was prepared according to a manufacturer's manual using Lipofectamine (TM) 2000 (Life technologies, Carlsbad, CA).

### Example 2: Intraarticular administration test of polyion complex-type micelles comprising mRNA

20 µL of polyion complex-type micelles prepared to comprise 3 µg of Luc2 was administered to the normal knee joints of ICR mice (8 week old) under anesthesia. 100 µL of a solution comprising 1.5 mg D-luciferin was intravenously administered to the mice, and the expression of luciferase in the joints was examined at various time points (24 hours, 48 hours, or 96 hours after the administration of micelles) using IVIS (TM) Imaging System (Xenogen, Alameda, CA, US).

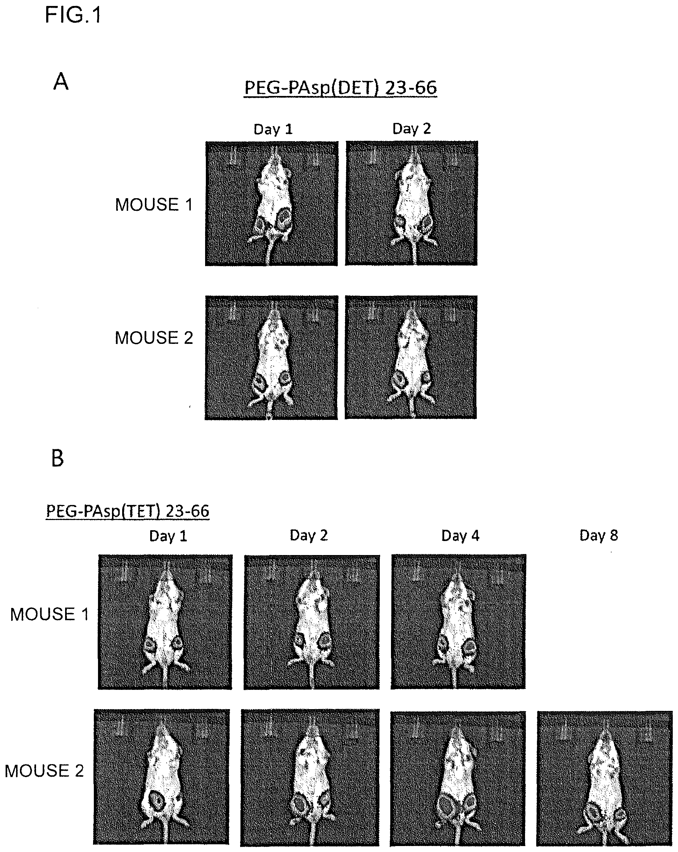

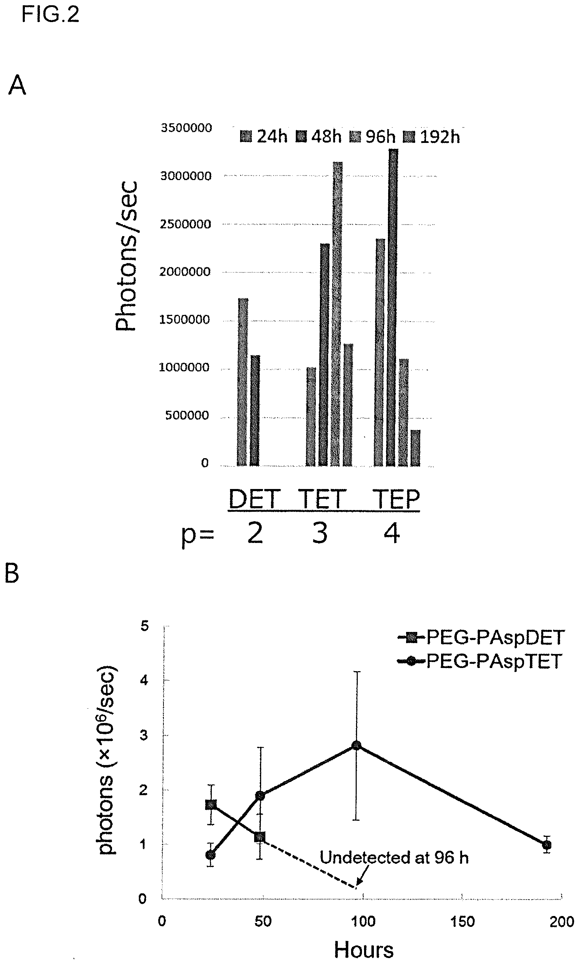

The expression of luciferase in the joints could be observed within one day after the intraarticular administration (Figures 1 and 2).

When PEG-PAsp (DET) was used as a cationic polymer, though the expression level decreased, the expression of luciferase was confirmed also 2 days after the administration (Figure 1A).

When PEG-PAsp (TET) was used as a cationic polymer, the expression was sustained for at least 4 days and the expression level was also maintained (Figure 1B). Though the expression level was decreased, the expression of luciferase was confirmed also 8 days after the administration (Figure 1B). For PEG-PAsp (TEP), photos are not shown, but the expression was sustained for at least 4 days as the same as PEG-PAsp (TET) and the expression of luciferase was confirmed even 8 days after the administration (data not shown).

The photon count per second was calculated from the obtained results to quantify the expression level of luciferase (Figure 2). According to Figures 2A and B, PEG-PAsp (DET) sustained the expression of mRNA for at least 2 days. PEG-PAsp (TET) highly expressed luciferase from one day after the administration, and surprisingly, it increased the expression level 2 days and 4 days after the administration, and sustained the expression for at least 8 days after the administration (Figure 2A and B). Further, PEG-PAsp (TEP) highly expressed luciferase 1 to 2 days after the administration, and then decreased the expression level, but sustained the expression for 8 days after the administration (Figure 2A).

Thus, all of PEG-PAsp (DET), PEG-PAsp (TET) and PEG-PAsp (TEP) could highly express mRNA in a living body for at least 2 days. PEG-PAsp (TET) and PEG-PAsp (TEP) could highly express mRNA in a living body for at least 8 days.

Next, the delivery of mRNA to the cartilages by the cationic polymers used in the Example and the presence or absence of toxicity caused by the delivery were examined.

20 µL of polyion complex-type micelles comprising EGFP mRNA (3 µg) and PEG-PAsp (DET) was intraarticularly administered to normal knees of mice. As a control, 20 µL of phosphate buffer solution (PBS) was intraarticularly administered to normal knees of mice in the same way. The knees were subjected to a histological analysis 24 hours after the administration.

Histological sections of the knees were prepared, and the expression of EGFP mRNA in the knee joints was immunohistologically examined using anti-GFP antibodies. Then, EGFP protein was expressed in the surface and the central part of the articular cartilage. The degree of cell death in the articular cartilage was conventionally observed using TUNEL method, which proved that there was no difference between the polyion complex-type micelle treated group and the PBS treated group. Thus, it was proved that the polyion complex-type micelle used in this Example could express mRNA in a living body (for example, in a knee joint) and that the administration in an amount allowing the expression of mRNA did not exhibit toxicity. Though mRNA is known to have high immunogenicity, problems resulting from the antigenicity of mRNA were not seen as far as we observed.

### Example 3: Treatment of osteoarthritis in osteoarthritis model animals

To date, delivery of mRNA into a living body and sustained expression thereof have been difficult, and thus there were high barriers for the treatment of diseases in a living body. According to Example 2, use of the polyion complex of the present invention allowed mRNA to be highly expressed in a living body sustainably for a long time. Therefore, in this Example, treatment of diseases using the polyion complex of the present invention was attempted.

As osteoarthritis model animals, osteoarthritis model mice were used. These model mice were prepared by surgically removing medial collateral ligaments and medial menisci of the knees (refer to S. Kamekura et al., Osteoarthritis Cartilage 13, 632-641 (2005)). Osteoarthritis was induced one month after the surgery. Seriousness of osteoarthritis was evaluated by the histological OARSI scoring system (see S. S. Glasson et al., 2010 Osteoarthritis Research Society International. Published by Elsevier Ltd, England, vol. 18 Suppl 3, pp. S17-23 (2010)).

Runx1 was selected as a factor promoting joint formation, and Runx1 mRNA and PEG-PAsp (DET) were mixed to form polyion complex-type micelles as described in Example 1. 20 µL of the obtained micelle solution (i.e., 3 µg as Runx1 mRNA) was administered to knees of the osteoarthritis mice one month after the surgery at a frequency of once every 3 days for one month. As a negative control, the polyion complex-type micelles in which EGFP was used as mRNA were administered to knees of the osteoarthritis mice one month after the surgery at a frequency of once every 3 days for one month.

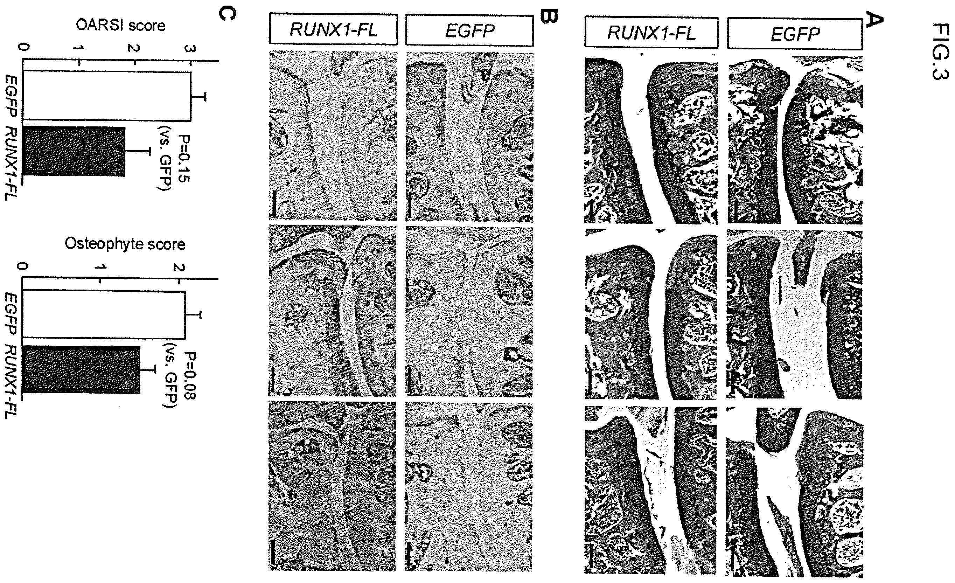

One month after the administration, typical symptoms of osteoarthritis were observed in tissue sections stained with safranin -O of the negative control. Observed symptoms included osteophyte and cartilage degradation, and these were proved by the reduction of the thickness of articular cartilage, staining intensity, and the destruction of the structure of articular cartilage (Figure 3A). On the other hand, in the group to which Runx1 mRNA was introduced, the phenotype of osteoarthritis tended to be inhibited, and in particular, the inhibition was significant in the osteophyte formation (Figure 3A).

The expression of the Runx1 protein in the knees two months after the surgery (i.e. one month after the completion of administration) was immunohistologically examined. The mice were euthanized and were fixed by circulating PBS including 4% paraformaldehyde. The knee joints were collected and further fixed overnight with PBS including 4% paraformaldehyde, and decalcified with PBS including 0.5 M ethylenediaminetetraacetic acid (EDTA). Then, the knee joints were embedded with paraffin to produce front sections with a thickness of 7 µm.

The sections were stained by combining an anti-Flag antibody (1:100; F1804, M2, Sigma-Aldrich), an anti-GFP antibody (1:500; ab290, Abcam, Cambridge, MA), an anti-Runx1 antibody (1:100; ab23980, Abcam), an anti-Sox9 antibody (1:200; sc20095, Santa Cruz Biotechnology), and an anti-PCNA antibody (1:1000; 2586s, Cell Signaling Technology) with CSAII Biotin-free Tyramide Signal Amplication System (Dako, Glostrup, Denmark). The sections were also stained with an anti-type X collagen antibody (1:500; LB-0092, LSL-Cosmo Bio co., ltd., Tokyo, Japan), an anti-type II collagen antibody (1:500; LB-1297, LSL-Cosmo Bio co., ltd.), an anti-IL-1β antibody (1:100; sc7884, Santa Cruz Biotechnology), and an anti-MMP-13 antibody (1:500; mab13426, Millipore). All sections were counterstained with methyl green.

The result showed that the Runx1 protein was strongly expressed in the articular cartilage and the expression was particularly significant in the osteophyte-like region (Figure 3B). The evaluation of the seriousness of arthropathy proved that the seriousness of group receiving administration of Runx1 mRNA decreased compared to the negative control group (Figure 3C).

Next, treatment of osteoarthritis was attempted as same as described above except that PEG-PAsp (TET) was used instead of PEG-PAsp (DET) as a cationic polymer.

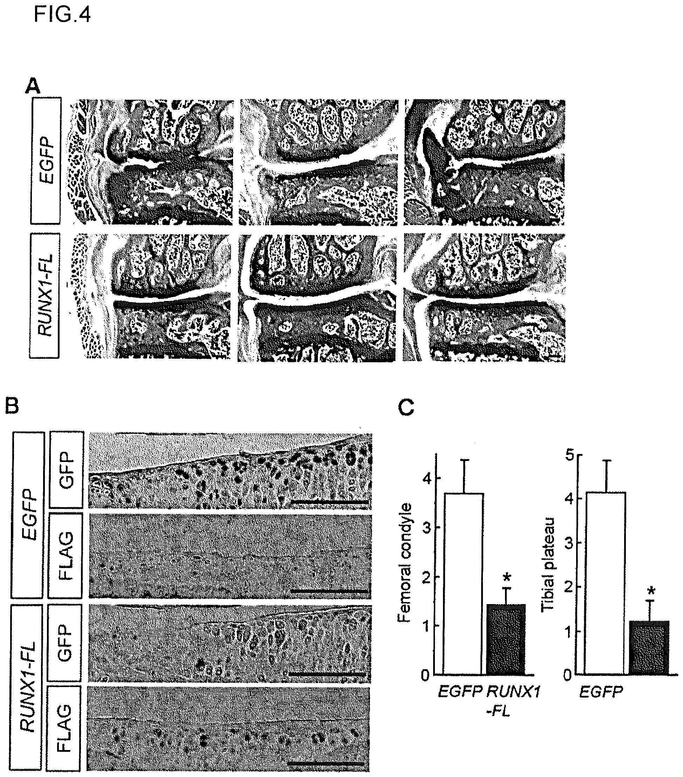

It was proved from the sections stained with safranin -O that osteoarthritis was inhibited in the group receiving administration of Runx1 mRNA compared to the EGFP-administered control group (Figure 4A). Expression of the Runx1 protein was strongly observed in the articular cartilage (data not shown). Further, the result of immunohistological observation showed that EGFP and Runx1 proteins were strongly expressed in the articular cartilage (Figure 4B). Osteoarthritis and the formation of osteophyte were statistically significantly inhibited in the group receiving administration of Runx1 mRNA compared to the negative control group (p < 0.05) (Figure 4C).

Further, expression levels of various factors were examined in the group receiving administration of Runx1 mRNA and the negative control group.

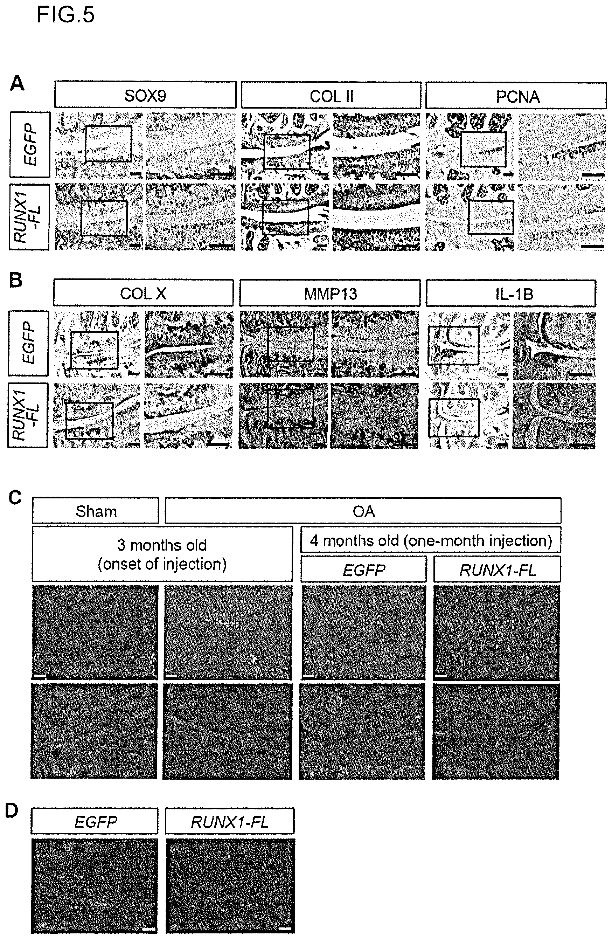

Sox9 is a master transcription factor of differentiation into cartilage and maintenance thereof in embryos and adults, and it was more strongly expressed in articular chondrocytes of the group receiving administration of Runx1 mRNA than the negative control group (upper left panels of Figure 5A). Type II collagen is a major cartilage matrix protein, and it was more strongly expressed in the group receiving administration of Runx1 mRNA than the negative control group (upper middle panels of Figure 5A). Further, proliferating cell nuclear antigen (PCNA) was also more strongly expressed in the group receiving administration of Runx1 mRNA than the negative control group (upper right panels of Figure 5). These results suggest that Runx1 has a function which promotes the early differentiation of chondrocytes.

On the other hand, the expression of type X collagen and MMP13 was not significantly different between the group receiving administration of Runx1 mRNA and the negative control group (lower left panels and lower middle panels of Figure 5). The expression level of IL-1β was lower in the group receiving administration of Runx1 mRNA than the negative control group (lower right panels of Figure 5).

Influence of administration of Runx1 mRNA on apoptosis was analyzed by TUNEL method. However, the cell death in the joints of osteoarthritis was not significantly different between the group receiving administration of the mRNA of Runx1 and the negative control group (Figure 5C and D).

Relation between rheumatoid arthritis and Runx1 is known and it is also known that pathological conditions are mediated by IL-1β in rheumatoid arthritis. In this Example, Runx1 significantly inhibited the expression of IL-1β in the joints, and thus it is apparent that rheumatoid arthritis can also be treated by the method of this Example.

## Claims

1. A polyion complex comprising a cationic polymer and mRNA for use in a method of treating osteoarthritis or rheumatoid arthritis,
wherein the cationic polymer is a polymer comprising a cationic unnatural amino acid as a monomer unit and the cationic unnatural amino acid is an amino acid having a group represented by -(NH-(CH₂)₂)ₚ-NH₂, wherein p is 2, 3 or 4, as a side chain.

2. The polyion complex for use according to claim 1, wherein the cationic polymer is a block copolymer with polyethylene glycol.

3. The polyion complex for use according to claim 1 or 2, wherein the cationic polymer is represented by the following general formula (I): 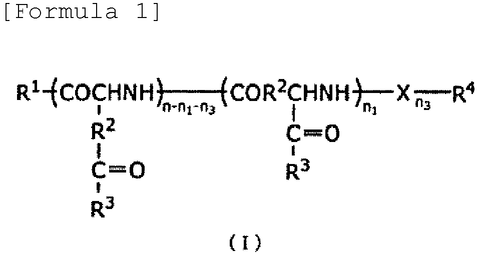 wherein
R¹ is polyethylene glycol and may be linked to an amino acid neighboring the polyethylene glycol through a linker,
R² is methylene or ethylene,
R³ is a group represented by - (NH- (CH₂)₂)ₚ-NH₂, and p is 2, 3 or 4,
R⁴ is hydrogen, a protecting group, a hydrophobic group,
or a polymerizable group,
X is a cationic natural amino acid,
n is any integer from 2 to 5000,
n₁ is any integer from 0 to 5000,
n₃ is any integer from 0 to 5000,
n - n₁ - n₃ is an integer equal to or greater than 0, and though the repeating units in the formula are shown in a particular order for convenience of description, the repeating units may be present in any order and the repeating units may be present at random, and each of the repeating units may be same or different.

4. A pharmaceutical composition for use in a method of treating osteoarthritis or rheumatoid arthritis comprising the polyion complex according to any one of claims 1 to 3,
wherein the mRNA is an mRNA of a factor promoting joint formation selected from Runt-related transcription factor 1 (Runx1), core-binding factor beta (Cbfbeta), sex-determining box 9 (Sox9) and proteoglycan 4.

5. The pharmaceutical composition for use according to claim 4, wherein p is 3 or 4.

6. The pharmaceutical composition for use according to claim 5, wherein p is 4.

7. The pharmaceutical composition for use according to any one of claims 4 to 6, wherein the pharmaceutical composition is administered once every 2 days or once every 3 days.

8. The pharmaceutical composition for use according to claim 5 or 6, wherein the pharmaceutical composition is administered once every 7 days.

9. A delivery agent for use in delivering mRNA into cells in a method of treating osteoarthritis or rheumatoid arthritis, said delivery agent comprising the polyion complex according to claim 3, wherein p is 3 or 4.

10. The delivery agent for use according to claim 9, wherein p is 4.

11. An in vitro method for delivering mRNA into cells, comprising the polyion complex according to any one of claims 1-3, wherein p is 3 or 4, wherein the mRNA is an mRNA of a factor promoting joint formation selected from Runt-related transcription factor 1 (Runx1), core-binding factor beta (Cbfbeta), sex-determining box 9 (Sox9) and proteoglycan 4.

## Patentansprüche

1. Polyionen-Komplex, umfassend ein kationisches Polymer und mRNA, zur Verwendung in einem Verfahren zur Behandlung von Osteoarthritis oder rheumatoider Arthritis,
wobei das kationische Polymer ein Polymer ist, welches eine kationische nicht-natürliche Aminosäure als Monomereinheit umfasst, und die kationische nicht-natürliche Aminosäure eine Aminosäure mit einer Gruppe,
repräsentiert durch -(NH-(CH₂)₂)ₚ-NH₂, wobei p 2, 3 oder 4 ist, als Seitenkette ist.

2. Polyionen-Komplex zur Verwendung gemäß Anspruch 1, wobei das kationische Polymer ein Block-Copolymer mit Polyethylenglycol ist.

3. Polyionen-Komplex zur Verwendung gemäß Anspruch 1 oder 2, wobei das kationische Polymer durch die folgende allgemeine Formel (I) repräsentiert ist: 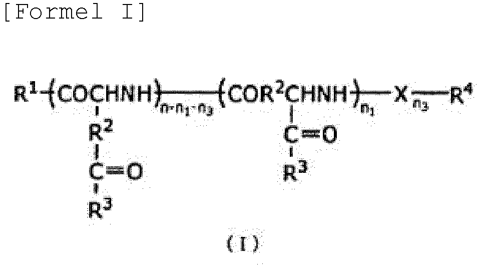 worin
R¹ Polyethylenglycol ist und mit einer zu dem Polyethylenglycol benachbarten Aminosäure durch einen Linker verknüpft sein kann,
R² Methylen oder Ethylen ist,
R³ eine Gruppe, repräsentiert durch -(NH-(CH₂)₂)ₚ-NH₂, ist und p 2, 3 oder 4 ist,
R⁴ Wasserstoff, eine Schutzgruppe, eine hydrophobe Gruppe oder eine polymerisierbare Gruppe ist,
X eine kationische natürliche Aminosäure ist,
n irgendeine ganze Zahl von 2 bis 5000 ist,
n₁ irgendeine ganze Zahl von 0 bis 5000 ist,
n₃ irgendeine ganze Zahl von 0 bis 5000 ist,
n-n₁-n₃ eine ganz Zahl gleich oder größer als 0 ist, und auch wenn die Wiederholungseinheiten in der Formel für die Einfachheit der Beschreibung in einer bestimmten Ordnung gezeigt sind, können die Wiederholungseinheiten in irgendeiner Ordnung vorliegen und die Wiederholungseinheiten können nach dem Zufallsprinzip vorliegen, und jede der Wiederholungseinheiten kann gleich oder unterschiedlich sein.

4. Pharmazeutische Zusammensetzung zur Verwendung in einem Verfahren zur Behandlung von Osteoarthritis oder rheumatoider Arthritis, welche den Polyionen-Komplex gemäß irgendeinem der Ansprüche 1 bis 3 umfasst,
wobei die mRNA eine mRNA eines die Gelenkbildung fördernden Faktors ist, ausgewählt aus mit Runt in Zusammenhang stehendem Transkriptionsfaktor 1 (Runx1), Kernbindungsfaktor beta (core-binding factor beta; Cbfbeta), geschlechtsbestimmender Box 9 (sex-determining box 9; Sox9) und Proteoglycan 4.

5. Pharmazeutische Zusammensetzung zur Verwendung gemäß Anspruch 4, wobei p 3 oder 4 ist.

6. Pharmazeutische Zusammensetzung zur Verwendung gemäß Anspruch 5, wobei p 4 ist.

7. Pharmazeutische Zusammensetzung zur Verwendung gemäß irgendeinem der Ansprüche 4 bis 6, wobei die pharmazeutische Zusammensetzung einmal alle 2 Tage oder einmal alle 3 Tage verabreicht wird.

8. Pharmazeutische Zusammensetzung zur Verwendung gemäß Anspruch 5 oder 6, wobei die pharmazeutische Zusammensetzung einmal alle 7 Tage verabreicht wird.

9. Zuführungsagens zur Verwendung beim Zuführen von mRNA in Zellen in einem Verfahren zur Behandlung von Osteoarthritis oder rheumatoider Arthritis, wobei das Zuführungsagens den Polyionen-Komplex gemäß Anspruch 3 umfasst, wobei p 3 oder 4 ist.

10. Zuführungsagens zur Verwendung gemäß Anspruch 9, wobei p 4 ist.

11. *In vitro*-Verfahren zum Zuführen von mRNA in Zellen, umfassend den Polyionen-Komplex gemäß irgendeinem der Ansprüche 1-3, wobei p 3 oder 4 ist, wobei die mRNA eine mRNA eines die Gelenkbildung fördernden Faktors ist, ausgewählt aus mit Runt in Zusammenhang stehendem Transkriptionsfaktor 1 (Runx1), Kernbindungsfaktor beta (core-binding factor beta; Cbfbeta), geschlechtsbestimmender Box 9 (sex-determining box 9; Sox9) und Proteoglycan 4.

## Revendications

1. Complexe polyionique comprenant un polymère cationique et un ARNm destiné à être utilisé dans un procédé de traitement de l'arthrose ou de la polyarthrite rhumatoïde,
dans lequel le polymère cationique est un polymère comprenant un acide aminé cationique non naturel en tant qu'unité monomère et l'acide aminé cationique non naturel est un acide aminé présentant un groupe représenté par-(NH-(CH₂)₂)ₚ-NH₂, dans lequel p vaut 2, 3 ou 4, en tant que chaîne latérale.

2. Complexe polyionique destiné à être utilisé selon la revendication 1, dans lequel le polymère cationique est un copolymère bloc avec du polyéthylène glycol.

3. Complexe polyionique destiné à être utilisé selon la revendication 1 ou 2, dans lequel le polymère cationique est représenté par la formule générale (I) suivante : 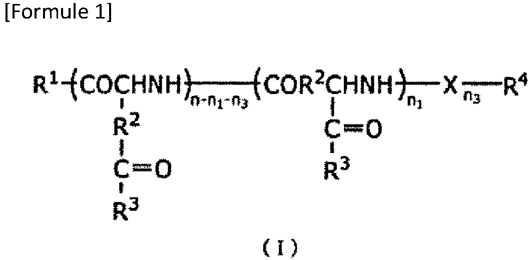 dans lequel
R¹ est un polyéthylène glycol et peut être lié à un acide aminé avoisinant le polyéthylène glycol par un lieur,
R² est un méthylène ou un éthylène,
R³ est un groupe représenté par -(NH-(CH₂)₂)ₚ-NH₂, et p vaut 2, 3 ou 4,
R⁴ est un hydrogène, un groupe protecteur, un groupe hydrophobe ou un groupe polymérisable,
X est un acide aminé cationique naturel,
n est tout nombre entier allant de 2 à 5 000,
n₁ est tout nombre entier allant de 0 à 5 000,
n₃ est tout nombre entier allant de 0 à 5 000,
n - n₁ - n₃ est un nombre entier supérieur ou égal à 0, et bien que les unités répétitives de la formule soient montrées selon un ordre particulier pour la facilité de la description, les unités répétitives peuvent être présentes quel que soit l'ordre et les unités répétitives peuvent être présentes de manière aléatoire, et chacune des unités répétitives peut être identique ou différente.

4. Composition pharmaceutique destinée à être utilisée dans un procédé de traitement de l'arthrose ou de la polyarthrite rhumatoïde comprenant le complexe polyionique selon l'une quelconque des revendications 1 à 3,
dans laquelle l'ARNm est un ARNm d'un facteur favorisant la formation articulaire choisi parmi le facteur de transcription lié Runt-1 (Runx1), le facteur de transcription hétérodimérique (Cbfbeta), le gène de détermination du sexe 9 (Sox9) et le protéoglycane 4.

5. Composition pharmaceutique destinée à être utilisée selon la revendication 4, dans laquelle p vaut 3 ou 4.

6. Composition pharmaceutique destinée à être utilisée selon la revendication 5, dans laquelle p vaut 4.

7. Composition pharmaceutique destinée à être utilisée selon l'une quelconque des revendications 4 à 6, dans laquelle la composition pharmaceutique est administrée une fois tous les 2 jours ou une fois tous les 3 jours.

8. Composition pharmaceutique destinée à être utilisée selon la revendication 5 ou 6, dans laquelle la composition pharmaceutique est administrée une fois tous les 7 jours.

9. Agent d'administration destiné à être utilisé lors de l'administration de l'ARNm dans des cellules dans un procédé de traitement de l'arthrose ou de la polyarthrite rhumatoïde, ledit agent d'administration comprenant le complexe polyionique selon la revendication 3, dans lequel p vaut 3 ou 4.

10. Agent d'administration destiné à être utilisé selon la revendication 9, dans lequel p vaut 4.

11. Procédé *in vitro* destiné à l'administration de l'ARNm dans des cellules, comprenant le complexe polyionique selon l'une quelconque des revendications 1-3, dans lequel p vaut 3 ou 4, dans lequel l'ARNm est un ARNm d'un facteur favorisant la formation articulaire choisi parmi le facteur de transcription lié Runt-1 (Runx1), le facteur de transcription hétérodimérique (Cbfbeta), le gène de détermination du sexe 9 (Sox9) et le protéoglycane 4.
